# EUROPEAN PATENT APPLICATION

(11) **EP 3 549 937 A1**
(43) Date of publication of application: **09.10.2019**
(21) Application number: 18165807.1
(22) Date of filing: 05.04.2018
(51) Int. Cl.: C07D 307/60

(54) **PROCESS FOR THE CONTINUOUS PREPARATION OF C16-ASA (HEXADECENE SUCCINIC ANHYDRIDE) AND/OR C18-ASA (OCTADECENE SUCCINIC ANHYDRIDE)**

(71) Applicant: Elsässer, Karl-Heinz, 40667 Meerbusch (DE); Wagener, Bärbel, 40667 Meerbusch (DE)
(72) Inventor: Elsässer, Karl-Heinz, 40667 Meerbusch (DE)
(74) Representative: Gille Hrabal

(57) **Abstract**

The present invention relates to a continuous process for the preparation of a succinic compound, selected from the group consisting of C₁₆-ASA (hexadecene succinic anhydride), C₁₈-ASA (octadecene succinic anhydride) and mixtures thereof.

## Description

The present invention relates to a process for the preparation of hexadecene succinic anhydride and octadecene succinic anhydride or mixtures thereof.

C₁₆-ASA (Hexadecene succinic anhydride) and C₁₈-ASA (octadecene succinic anhydride) are commonly used as sizing agents in the alkaline production of paper to improve the quality of the paper (e.g. writing properties). Other applications for hexadecene succinic anhydride and octadecene succinic anhydride include the use as surfactant intermediate, engine oil lubricant additive, viscosity and pour-point improver, plasticizer, epoxy, antifreeze lubricant, moisture control for leather goods and corrosion inhibitor.

Hexadecene succinic anhydride and octadecene succinic anhydride are comparatively expensive chemical compounds due to the production pathways used until today. Usually, hexadecene succinic anhydride and octadecene succinic anhydride are prepared from the corresponding alkene and maleic anhydride in a batch reactor at temperatures of 200 to 230 °C with a reaction time of around 8 hours at atmosphere pressure under inert conditions according to the following reaction scheme (for octadecene succinic anhydride):

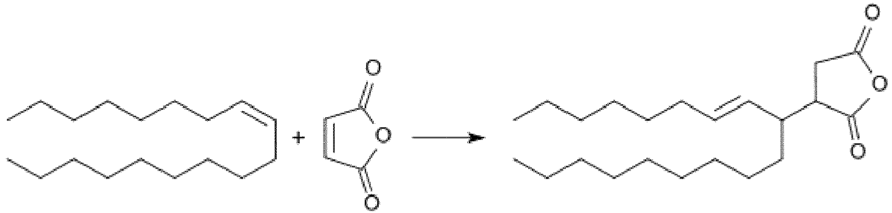

The reaction scheme for hexadecene succinic anhydride is similar.

Since maleic anhydride is almost insoluble in the alkene compound and usually no solvent is used, the resulting liquid two-phase system of the alkene and the maleic anhydride requires the above-mentioned very long reaction times (around 8 hours) and temperatures (200 to 230 °C).

One challenge of the preparation procedure is determined by the potential risk of self-ignition and combustion of the alkene compound by temperatures above 240 °C. Accordingly, the use of higher temperatures as 230 °C should be avoided.

The reaction of the alkene compound with the maleic anhydride is usually incomplete requiring a distillation of the alkene compound together with the maleic anhydride under vacuum. The unreacted alkene compound is taken out of the distillation and decanted, whereby the alkene compound is recycled into the reaction vessel and the maleic anhydride is dismissed.

The product of the reaction - the alkene succinic compound - is provided at the bottom of the reaction vessel, filtered to remove solid side-products and isolated. Moreover, some of the succinic product is also removed by the distillation and returned into the reaction vessel along with the alkene compound. The succinic product from the top stream, which is provided back into the reaction vessel, underlies a further thermal stress resulting in even higher decomposition rates of the succinic product.

The reaction requires, as already mentioned above, relative high temperatures with a rather long residence time in the reaction vessel. This procedure provides a huge amount of side-products generated, for example, by enophilic polymerizations, alkene oligomerizations, copolymerizations between the enophile and the alkene compound, and partial decompositions of the succinic products by a retro-ene reaction.

The side-products thus generated are usually dark solids and disturb the intended use of the succinic products, in particular in case the succinic compound is used as sizing agent in the paper production. Moreover, the yield of the succinic compound is reduced by the formation of the afore-mentioned side-products.

In the prior art, there are a few processes described which try to reduce the formation of side-products during the preparation of C₁₆-ASA (hexadecane succinic anhydride) and C₁₈-ASA (octadecene succinic anhydride), such as US 3,202,679; US 3,412,111; and US 3,819,660. In the processes described therein, antioxidants or polymerization inhibitors (hydrochinons and phenothiazines) are added into the reaction vessel in order to minimize the formation of side-products. However, the use of these antioxidants or polymerization inhibitors do not improve remarkably the yield of the succinic product, and the use of these antioxidants or polymerization inhibitors raises the costs for the overall process. Moreover , the color of the resulting succinic product is still not satisfying (Nuno F. Duarte, Joao P. Telo and Joao C. Bordado, *Synthesis and characterization of ASA samples*)*.* Finally, most of the produced succinic compounds are used in the technical field of paper sizing and the use of antioxidants or polymerization inhibitors does not comply with this technical field.

One further approach to improve the yield of the succinic compound is to use an excess of the starting material, in particular of the alkene compound, in order to shift the equilibrium to the product side. However, experiments show that the equilibrium between the alkene compound and the succinic compound can only slightly be influenced by the concentration of the starting material.

Moreover, the processes known from prior art have a poor ratio of the reaction time to the batch time resulting in low efficiency and poor space-time yield (the overall duration of the process of the prior art is pretty long (around 15 to 16 hours, including distillation)). Thus, the processes known from prior art are expensive and time-consuming.

Starting from this prior art situation, there is still need for a modified and improved process for the preparation of C₁₆- ASA (hexadecene succinic anhydride) and C₁₈-ASA (octadecene succinic anhydride) which is easy to carry out.

This process should preferably shorten the time necessary for providing hexadecene succinic anhydride and octadecene succinic anhydride and thereby providing a reduced amount of side-products.

Moreover, the intended process should provide the succinic compound with a genuine color and with a high yield. In addition, the process time and the space-time yield should be improved as compared with the processes known from prior art.

Finally, the intended process should allow a cheaper preparation of the succinic compound and should use easily available production facilities.

These objects are solved by a process for the preparation of a succinic compound, selected from the group consisting of C₁₆-ASA (hexadecene succinic anhydride), C₁₈-ASA (octadecene succinic anhydride) and mixtures thereof.

The claimed process is characterized in that an alkene compound, selected from the group consisting of hexadecene, octadecene or mixtures thereof, is continuously reacted with maleic anhydride in a reaction vessel and the hexadecene succinic anhydride and/or octadecene succinic anhydride thus prepared is removed out of the resulting reaction mixture by vacuum rectification.

Thus, the claimed invention proposes, for the first time, a continuous process for the preparation of the succinic compound, whereby the product is directly removed out of the reaction vessel by a rectification under vacuum providing higher yields and reduced side-products as compared with the previous known processes including a distillation under vacuum after a batch reaction.

Before the present invention is described in more detail, the following definitions are made:
The claimed process uses - as a starting material - hexadecene, octadecene or any suitable mixture thereof. These compounds have an internal double bond to which the maleic anhydride binds under the shift of the double bond. The respective hexadecene, octadecene or mixtures thereof are usually prepared by starting from normal α-C₁₆ or C₁₈ olefins by isomerization to a thermodynamic distribution of internal olefins. The isomerization is usually carried out by heating the α-olefin in the presence of a suitable catalyst to move the double bond from a terminal position of the olefin to an internal position. Typically, the isomerization process creates a complex mixture of various olefin isomers. The preparation of internal olefins by a metathesis reaction and the utility of the metathesized olefins in the preparation of alkenyl succinic anhydride (ASA) compounds are disclosed in US 2003/0224945 A. In the framework of the present invention hexadecene, octadecene and any suitable mixture thereof are abbreviated as alkene compound. These olefins have an internal double bond.

The claimed process provides - as the end product - C₁₆-ASA (hexadecene succinic anhydride), C₁₈-ASA (octadecene succinic anhydride) or mixtures thereof. In case the present invention provides mixtures of both succinic anhydrides the ratio between the C₁₆ and C₁₈ succinic anhydride depends from the ratio of the C₁₆ to C₁₈ alkene compounds used as starting material. In the framework of the present invention, hexadecene succinic anhydride, octadecene succinic anhydride or mixtures thereof are abbreviated as succinic compound. These succinic olefins have an internal double bond.

In the following, the reaction of the alkene compound with the maleic anhydride and the rectification are described separately.

### Reaction of the alkene compound with the maleic anhydride

The alkene compound is reacted with maleic anhydride.

This process step according to the present invention is preferably carried out in a manner that the mean residence time in the reaction vessel is adjusted so that at least 40 %, preferably at least 45 %, more preferably at least 50 %, more preferably at least 65 %, of the alkene component are converted into the succinic compound. In case this mean residence time is maintained, the overall yield of the succinic compound is improved and the amount of degraded alkene compound is minimized.

The mean residence time of the reaction mixture in the reaction vessel is adjusted by the amount of reaction mixture in the reaction vessel to withdrawn succinic compound out of the reaction vessel.

Usually, the mean residence time of the reaction mixture in the reaction vessel is preferably at least 4 hours, preferably at least 4.5 hours, more preferably at least 6 hours, more preferably at least 6.5 hours.

The amount of fresh alkene compound and maleic anhydride fed into the reaction vessel depends on the amount of succinic compound withdrawn from the reaction vessel through rectification and the amount of alkene compound and maleic anhydride recycled from the rectification.

The claimed process can be carried out in any suitable equipment, but it is preferred that the reaction vessel is a continuous stirred tank reactor. Such a reaction vessel is a usual facility in chemical production plants and can easily be modified for a combination with a rectification column.

Usually, the molar ratio of the alkene compound to the maleic anhydride in the reaction vessel is from 1 : 1 to 5 : 1, preferably 1 : 1 to 4: 1, more preferably 1 : 1 to 3 : 1, more preferably 1 : 1 to 2 : 1, whereby the amount of the alkene compound results from the effective amount of the alkene compound in the reaction vessel, the amount of recycled alkene compound and the amount of freshly added alkene compound and the amount of maleic anhydride results from the effective amount of maleic anhydride in the reaction vessel, the amount of recycled maleic anhydride and the amount of freshly added maleic anhydride.

The reaction between the alkene compound and maleic anhydride is carried out at a temperature of in general 170 to 240 °C, preferably 180 and 230 °C, more preferably 190 and 210 °C.

The reaction between the alkene compound and the maleic anhydride is carried out preferably with a molar ratio of the alkene compound to the maleic anhydride of essentially 1 : 1 and at a temperature of essentially 210 °C.

The reaction between the alkene compound and the maleic anhydride is usually carried out under inert conditions. Suitable inert gases are nitrogen or argon.

The reaction of the alkene compound with maleic anhydride is carried out in general at atmospheric pressure or overpressure. In case the reaction is carried out at an overpressure, the overpressure is usually at most 350 mbar, preferably at most 300 mbar, more preferably at most 275 mbar, more preferably at most 250 mbar.

### Rectification

One of the key elements of the process according to the present invention is the continuous conversion of the alkene compound with maleic anhydride to the succinic compound.

Accordingly, it is also preferred that the vacuum rectification is carried out continuously in order to comply with the continuous reaction of the alkene compound with maleic anhydride. The unreacted alkene compound and maleic anhydride is preferably removed from the top of the rectification column as a head product and the succinic compound, as a hard boiler, is removed from the bottom of the rectification column.

This head product, which comprises the unreacted alkene compound and maleic anhydride, is preferably recirculated into the reaction vessel. Since the rectification is carried out preferably in a continuous manner, it is also preferred to recirculate the alkene compound and the maleic anhydride continuously into the reaction vessel.

The succinic compound being removed from the bottom of the rectification column as bottom product usually does not require a further process step of purification. However, if necessary for specific purposes, the succinic compound thus prepared can be subjected to one or several purification steps.

The vacuum rectification is carried out under reduced pressure of from in general 20 mbar or less, preferably 15 mbar or less, more preferably 10 mbar or less, more preferably 5 mbar or less. A vacuum rectification under reduced pressure of less than 5 mbar provides the succinic compound with a very high purity. The succinic compound thus prepared has a purity of about 97 % or more.

The preparation costs for the succinic compound prepared according to the present invention can be reduced by up to 20 to 30 % as compared with the known batch processes.

### Example

The present invention will be described in detail with reference to the following figure and example:

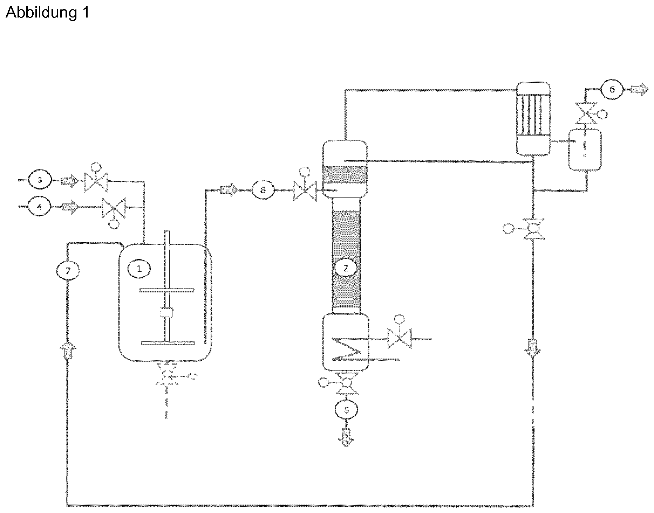

Figure 1 shows a flow chart for the claimed process. The claimed process is carried out in a continuously stirred tank reactor (1) which is combined with a rectification unit (2) processed under vacuum. Both, the stirred tank reactor (1) and the rectification unit (2) are working continuously.

The alkene compound, hexadecane, octadecene or a mixture of hexadecane and octadecene, is introduced via inlet (3) into the continuously stirred tank reactor (1), while maleic anhydride is inserted into the continuously stirred tank reactor (1) via the inlet (4). The succinic product, i.e. C₁₆-ASA (hexadecene succinic anhydride), C₁₈-ASA (octadecene succinic anhydride) or a mixture of C₁₆-ASA and C₁₆-ASA , is formed in the continuously stirred tank reactor (1) for the indicated time below under an inert atmosphere of nitrogen at 210 °C and atmospheric pressure. After the succinic compound is formed, the product is removed continuously from the rectification unit (2) via outlet (5) at the bottom of the rectification unit (2) as high boiler, whereas the unprocessed alkene compound along with the unreacted maleic anhydride are withdrawn from the top of the rectification column and fully and continuously recycled into the reaction vessel (1) via path (7). The rectification is carried out under reduced pressure of less than 5 mbar provided via pathway (6).

The process according to Figure 1 is carried out with 5 or 6 hours residence time and a conversion rate of 55 % (after 5 hours) or 65 % (after 6 hours).

The prepared succinic compound has a higher purity as compared with a succinic compound being prepared by using a batch procedure. Moreover, the process time is remarkably removed.

## Claims

1. A process for the preparation of a succinic compound, selected from the group consisting of hexadecene succinic anhydride, octadecene succinic anhydride and mixtures thereof,
**characterized in that**
an alkene compound, selected from the group consisting of hexadecene, octadecene or mixtures thereof, is continuously reacted with maleic anhydride in a reaction vessel and the succinic compound thus prepared is removed out of the resulting reaction mixture by vacuum rectification.

2. The process according to claim 1, **characterized in that** the vacuum rectification is carried out continuously.

3. The process according to claim 1 or 2, **characterized in that** the unreacted alkene compound and unreacted maleic anhydride is removed from the top of the rectification column as a head product and is recirculated into the reaction vessel.

4. The process according to anyone of claims 1 to 3, **characterized in that** the succinic compound is continuously removed from the bottom of the rectification column as a bottom product.

5. The process according to anyone of claims 1 to 4, **characterized in that** the mean residence time of the alkene compound in the reaction vessel is adjusted so that at least 40 % of the alkene component are converted into the succinic compound.

6. The process according to anyone of claims 1 to 5, **characterized in that** the mean residence time of the alkene compound in the reaction vessel is at least 4 hours.

7. The process according to anyone of claims 1 to 6, **characterized in that** the reaction vessel is a continuous stirred tank reactor.

8. The process according to anyone of claims 1 to 7, **characterized in that** the molar ratio of the alkene compound to the maleic anhydride in the reaction vessel is form 1 : 1 to 5 : 1.

9. The process according to anyone of claims 1 to 8, **characterized in that** the reaction between the alkene compound and maleic anhydride is carried out at a temperature of between 170 to 240 °C.

10. The process according to anyone of claims 1 to 9, **characterized in that** the reaction between the alkene compound and the maleic anhydride is carried out under inert conditions of a nitrogen or argon atmosphere.

11. The process according to anyone of claims 1 to 10, **characterized in that** the vacuum rectification is carried out at reduced pressure of from 20 mbar or less.

12. The process according to anyone of claims 1 to 11, **characterized in that** the reaction of the alkene compound with maleic anhydride in the reaction vessel is carried out by atmospheric pressure or overpressure.

13. The process according to anyone of claims 1 to 12, **characterized in that** the reaction of the alkene compound with maleic anhydride in the reaction vessel is carried out at an overpressure of at most 350 mbar.

14. The process according to any of claims 1 to 13, **characterized in that** the alkene compound is prepared from α-olefins by isomerization.
